# EUROPÄISCHE PATENTSCHRIFT

(11) **EP 3 286 312 B1**
(45) Veröffentlichungstag und Bekanntmachung des Hinweises auf die Patenterteilung: **20.07.2022**
(21) Anmeldenummer: 16711779.5
(22) Anmeldetag: 26.02.2016
(51) Int. Cl.: C12Q 1/6806

(54) **VORRICHTUNG UND VERFAHREN ZUR EXTRAKTION VON NUKLEINSÄUREN**
DEVICE AND METHOD FOR EXTRACTING NUCLEIC ACIDS
PROCÉDÉ ET DISPOSITIF D'EXTRACTION D'ACIDES NUCLÉIQUES

(30) Priorität: 23.04.2015 DE 102015207481; 19.06.2015 DE 102015211393; 19.06.2015 DE 102015211394
(43) Veröffentlichungstag der Anmeldung: 28.02.2018
(73) Patentinhaber: IST Innuscreen GmbH, 13125 Berlin (DE)
(72) Erfinder: HILLEBRAND, Timo, 15366 Hoppegarten (DE); STROH, Thorsten, 10713 Berlin (DE)
(74) Vertreter: Wehlan, Helmut
(86) Internationale Anmeldenummer: PCT/EP2016/054179
(87) Internationale Veröffentlichungsnummer: WO 2016/169678

(56) Entgegenhaltungen:
- DE-A1- 3 717 211
- AKONNI: "TruTip - Breaking the speed limit on ultra-rapid nucleic acid extraction", INTERNET CITATION, 16. November 2010 (2010-11-16), Seiten 1-8, XP002753497, Gefunden im Internet: URL:http://www.akonni.com/docs/TruTip%20Br ochure.pdf [gefunden am 2010-11-16]
- DARRELL P CHANDLER ET AL: "Rapid, simple influenza RNA extraction from nasopharyngeal samples", JOURNAL OF VIROLOGICAL METHODS, ELSEVIER BV, NL, Bd. 183, Nr. 1, 1. März 2012 (2012-03-01), Seiten 8-13, XP028483393, ISSN: 0166-0934, DOI: 10.1016/J.JVIROMET.2012.03.002 [gefunden am 2012-03-07]
- HOLMBERG R C ET AL: "High-throughput, automated extraction of DNA and RNA from clinical samples using TruTip technology on common liquid handling robots", JOVE, JOURNAL OF VISUALIZED EXPERIMENTS, JOURNAL OF VISUALIZED EXPERIMENTS, US, Nr. 76, 1. Juni 2013 (2013-06-01), Seiten e50356-1, XP002753498, ISSN: 1940-087X, DOI: 10.3791/50356 [gefunden am 2013-06-11]
- VOGELSTEIN B ET AL: "PREPARATIVE AND ANALYTICAL PURIFICATION OF DNA FROM AGAROSE", PROCEEDINGS OF THE NATIONAL ACADEMY OF SCIENCES, NATIONAL ACADEMY OF SCIENCES, US, Bd. 76, Nr. 2, 1. Februar 1979 (1979-02-01), Seiten 615-619, XP002906050, ISSN: 0027-8424, DOI: 10.1073/PNAS.76.2.615 in der Anmeldung erwähnt

## Beschreibung

Gegenstand der Erfindung ist eine neuartige Vorrichtung, mit der Nukleinsäuren schnell und hocheffizient sowie quantitativ isoliert oder aufgereinigt werden können. Die neuartige Vorrichtung zur Extraktion von Nukleinsäuren kann sowohl für eine manuelle Extraktion im Labor als auch unter Feldbedingungen eingesetzt werden. Besondere Vorteile offenbart es im Kontext mit der Automatisierung von Nukleinsäureextraktionen.

Unter klassischen Bedingungen erfolgt die Isolierung von DNA aus Zellen und Geweben dadurch, dass die Nukleinsäuren enthaltenden Ausgangsmaterialien unter stark denaturierenden und reduzierenden Bedingungen, teilweise auch unter Verwendung von proteinabbauenden Enzymen aufgeschlossen, die austretenden Nukleinsäurefraktionen über Phenol-/ Chloroform- Extraktionsschritte gereinigt und die Nukleinsäuren mittels Dialyse oder Ethanolpräzipitation aus der wässrigen Phase gewonnen werden (Sambrook, J., Fritsch, E.F. und Maniatis, T., 1989, CSH, "Molecular Cloning"). Diese "klassischen Verfahren" zur Isolierung von Nukleinsäuren aus Zellen und besonders aus Geweben sind sehr zeitaufwendig (teilweise länger als 48 h), erfordern einen erheblichen apparativen Aufwand und sind darüber hinaus auch nicht unter Feldbedingungen realisierbar. Außerdem sind solche Methoden auf Grund der verwendeten Chemikalien wie Phenol und Chloroform in einem nicht geringen Maße gesundheitsgefährdend.

Die nächste Verfahrensgeneration zur Isolierung von Nukleinsäuren basiert auf einer von Vogelstein und Gillespie (Proc. Natl. Acad. Sci. USA, 1979, 76, 615 - 619) entwickelten und erstmals beschriebenen Methode zur präparativen und analytischen Reinigung von DNA-Fragmenten aus Agarosegelen. Die Methode kombiniert die Auflösung der die zu isolierende DNA- Bande enthaltenden Agarose in einer gesättigten Lösung eines chaotropen Salzes (NaJ) mit einer Bindung der DNA an Glaspartikel. Die an die Glaspartikel fixierte DNA wird anschließend mit einer Waschlösung (20 mM Tris HCl [pH 7,2]; 200mM NaCl; 2 mM EDTA; 50% v/v Ethanol) gewaschen und danach von den Trägerpartikeln abgelöst. Diese Methode erfuhr bis heute eine Reihe von Modifikationen und wird zum gegenwärtigen Zeitpunkt für unterschiedliche Verfahren der Extraktion und Reinigung von Nukleinsäuren aus unterschiedlichen Herkünften angewendet und ist letztlich die Basis für fast alle kommerziell verfügbaren Kits zur manuellen wie auch automatisierten Isolierung von Nukleinsäuren. Darüber hinaus gibt es eine Vielzahl von Patenten und Veröffentlichungen, die sich auf das Basisprinzip der Isolierung von Nukleinsäuren beziehen, welches von Vogelstein und Gillespie erstmals publiziert wurde und ggf. weitere Vorteile innehaben. Diese Varianten betreffen sowohl die Verwendung unterschiedlicher mineralischer Trägermaterialien als auch die Art der für die Bindung der Nukleinsäuren eingesetzten Puffer. Beispiele sind u.a. die Bindung von Nukleinsäuren an mineralische Träger unter Anwesenheit von Lösungen unterschiedlicher chaotroper Salze, bei welchen als Trägermaterial feingemahlene Glaspulver (BIO 101, La Jolla, CA), Diatomenerden (Fa. Sigma) oder auch Silicagele bzw. Silcasuspensionen oder Glasfaserfilter oder mineralische Erden (DE 41 39 664 A1; US 5,234,809; WO-A 95/34569 DE 4321904; DE 20207793) zum Einsatz kommen. All diese Patente basieren auf der Anbindung von Nukleinsäuren an ein mineralisches Trägermaterial auf der Basis von Glas oder Silizium unter Anwesenheit chaotroper Salzlösungen. In neueren Patentschriften wird offenbart, dass für die Adsorption von Nukleinsäuren an die dem Fachmann bekannten und eingesetzten mineralischen Materialien auch sogenannte antichaotrope Salze als Bestandteil von Lyse-/ Bindungspuffer- Systemen sehr effizient und erfolgreich eingesetzt werden können (EP 1135479). Zusammenfassend kann man den Stand der Technik also dahingehend beschreiben, dass Nukleinsäuren an mineralische Materialien in Anwesenheit von Puffern, die chaotrope oder antichaotrope Salze enthalten oder auch in Anwesenheit von Puffern die Mischungen chaotroper und antichaotroper Salze enthalten, binden und auf diesem Wege dann auch isoliert werden können. Dabei gibt es auch Vorzugsvarianten, bei denen zusätzlich aliphatische Alkohole zur Bindungsvermittlung eingesetzt werden. Dem Fachmann ist auch bekannt, dass alle gängigen kommerziellen Produkte zur Isolierung und Aufreinigung von Nukleinsäuren auf dieser Grundlage basieren. Die eingesetzten mineralischen Träger liegen dabei in Form von losen Schüttungen, in Form von Filtermembranen oder auch in Form von Suspensionen vor. Für die Durchführung von automatisierten Extraktionsabläufen werden häufig paramagnetische oder magnetische Partikel eingesetzt. Dabei handelt es sich z.B. um silicatische Materialien, die einen magnetischen oder paramagnetischen Kern besitzen oder aber auch um Eisenoxydpartikel, deren Oberfläche so modifiziert wird, dass diese die für die Anbindung der Nukleinsäuren notwendigen Funktionalitäten tragen. Um insbesondere automatisierte Extraktionen einfacher durchführen zu können, wurden modifizierte Pipettenspitzen eingesetzt. Diese sind dadurch charakterisiert, dass sie die zur Anbindung von Nukleinsäuren notwendigen Trägermaterialien (poröse mineralische Trägermaterialien oder poröse Anionenaustauscher etc.) bereits enthalten. So beschreibt die Patentschrift DE3717211 eine Pipettenspitze mit einem porösen Chromatographiematerial für die Isolierung von Nukleinsäuren. Die Patentschrift EP1951904 offenbart eine Pipettenspitze, bestehend aus einem Ober-und Unterteil, zwischen welchem sich ebenfalls ein poröses chromatographisches Trägermaterial befindet und welche für die automatisierte Isolierung von Nukleinsäuren eingesetzt werden soll. Eine modifizierte Pipettenspitze zur Extraktion von Nukleinsäuren ist auch in der Offenlegungsschrift US2013/0078619 offenbart. Auch in dieser Pipettenspitze befindet sich ein poröses mineralisches Trägermaterial (poröses Glas) zur direkten Anbindung von Nukleinsäuren. All diesen modifizierten Pipettenspitzen ist gemeinsam, dass es sich um ein poröses chromatographisches Material handelt (lose Schüttung oder fester poröser Körper). Diese Trägermaterialien befinden sich immer horizontal innerhalb der Pipettenspitze. Die zu prozessierenden Flüssigkeiten strömen durch das eingesetzte poröse Material. Der Extraktionsablauf basiert darauf, dass nach Lyse der Probe und Einstellung notwendiger Bindungsbedingungen für die Adsorption der Nukleinsäuren an das Trägermaterial, dieser Ansatz mittels eines Pipettiervorganges durch das poröse Trägermaterial gezogen wird. Die Nukleinsäuren binden an das Trägermaterial. Nachfolgend werden Waschpuffer durch das Trägermaterial pipettiert. Danach erfolgt ein Trocknungsschritt (häufiges Auf-und Abpipettieren oder Anlegen von Vakuum). Final wird das Elutionsmittel durch das Trägermaterial pipettiert. Dabei wird die gebundene Nukleinsäure vom Trägermaterial abgelöst. Die Verwendung von Trägermaterial enthaltenden Pipettenspitzen soll den (insbesondere) automatisierten Ablauf von Nukleinsäureextraktionen deutlich vereinfachen. Obwohl diese Ideen schon teilweise relativ alt sind (die Patentschrift DE3717211 datiert vom 22.5.1987), hat sich ein solches Verfahren nicht durchgesetzt. Die Ursache liegt dabei in einigen grundsätzlichen Problemen:
1) Das Pipettieren von Nukleinsäure enthaltenden Lysaten hoher Viskosität funktioniert nur eingeschränkt oder führt zum vollständigen Verschluss des chromatographischen Materials. Damit ist keine Extraktion möglich.
2) Das Pipettieren von Lysaten über ein poröses Material führt zur Schaumbildung. Dieses wird mit der zunehmenden Anzahl von Pipettierschritten verstärkt und kann den Extraktionsprozess ebenfalls unmöglich machen.
3) Die Entfernung von alkoholischen Komponenten aus einem porösen Material ist schwierig und ist oftmals nicht zufriedenstellend gelöst.

Zum Stand der Technik gehört auch die Offenlegungsschrift WO 01/05510 A1. Darin wird ein Hohlkörper beschrieben, der magnetische Partikel enthält. Über die Oberflächeneigenschaften dieser Magnetpartikel werden keine Angaben gemacht. Die Nukleinsäureanbindung mittels Magnetpartikeln erfolgt normalerweise mittels glatter Eisenpartikel. Die Oberflächeneigenschaften spielten im Stand der Technik keine Rolle, nur die magnetischen Eigenschaften.

Der Erfindung lag deshalb die Aufgabe zu Grunde, die bekannten Probleme zu lösen und ein einfaches und schnelles Verfahren der Extraktion von Nukleinsäuren mittels einer modifizierten Pipettenspitze zu ermöglichen.

Die Aufgabe wurde gemäß den Merkmalen der Patentansprüche gelöst. Gemäß Anspruch 1 wird eine Vorrichtung zur Extraktion von Nukleinsäuren bereitgestellt, umfassend einen Hohlkörper, durch den eine Flüssigkeit geleitet wird, wobei in diesem Hohlkörper ein Material in Form von Metall-Schrauben oder Metallschwämmen oder einer Scheibe aus angerautem Polymer oder angerauter Plastgranulate so angeordnet ist, dass es von einer Flüssigkeit umspült werden kann. Bei dem Material ist die Rauheit der Scheibe oder Plastgranulate durch Berühren oder durch Ansicht erkennbar. In einer bevorzugten Ausführungsform fungiert als Hohlkörper eine Pipettenspitze. Das Material hat eine solche Größe, dass es aus der Pipettenspitze unten nicht herausgelangen kann und unterscheidet sich damit von den im Stand der Technik beschriebenen magnetischen Partikeln (WO 01/05510 A1). Die Ansprüche 2 bis 3 beschreiben bevorzugte Ausführungsformen der Vorrichtung. Die Erfindung umfasst auch ein Gerät nach dem walk-away-Prinzip, welches die Vorrichtung verwendet. Außerdem wird ein Verfahren zur Isolierung von Nukleinsäuren mittels der Vorrichtung beschrieben. Dieses Verfahren ist durch folgende Schritte gekennzeichnet:
a) Eine lysierte biologische Probe wird mit mindestens mit einer Substanz, die die Polarität der wässrigen Lösung senkt oder mit einem Mittel zur Anbindung von Nukleinsäuren an eine feste Phase versetzt
b) Aufziehen dieser Mischung unter a) mit einer Pipettenspitze, in der sich ein Material gemäß einem der Ansprüche 1 bis 3 befindet und mehrmaliges Auf- und Abpipettieren, wobei sich die Flüssigkeit dabei am Material vorbei bewegt und die Nukleinsäuren an dem rauen oder strukturierten Material präzipitieren - und damit an die feste Phase angebunden werden
c) Entfernen der Pipettenspitze aus der Probe
d) Eintauchen der Pipettenspitze in eine Waschpufferlösung und mehrmaliges Auf- und Abpipettieren, wobei sich die Flüssigkeit dabei am Material vorbei bewegt.
e) Trocknung der Pipettenspitze zur Entfernung des restlichen Alkohols vom Waschpuffer
f) Ablösen der Nukleinsäure mit einem Elutionspuffer durch mehrmaliges Auf- und Abpipettieren des Elutionspuffers, wobei sich der Elutionspuffer dabei am Material vorbei bewegt.

Überraschender Weise kann dies mit einfachen Mitteln erreicht werden. Es wurde entdeckt, dass man ein nukleinsäurebindendes Material nicht horizontal in eine Pipettenspitze verbringt, sondern vertikal, so dass an einer oder an beiden Seiten des nukleinsäurebindenden Materials die Flüssigkeit unbehindert vorbeifließen kann. In einer anderen Ausführungsform kann die Pipettenspitze auch mit einem nukleinsäurebindenden partikulärem Material der Art befüllt sein, das sich zwischen diesem Material ausreichend große Hohlräume befinden, so dass eine Flüssigkeit ebenfalls an diesem Material verbeifließt und nicht durch dieses Material hindurch. Eine weitere mögliche Ausführungsform besteht darin, dass in eine Pipettenspitze sich ein oberflächenstrukturiertes Material befindet. In diesem Fall fließt die Flüssigkeit ebenfalls an den "Strukturen" des Materials vorbei. Alle diese Ausführungsformen bedeuten letztlich, dass die für die Isolierung von Nukleinsäuren eingesetzten Flüssigkeiten nicht durch ein chromatographisches Material hindurch, sondern an einem nukleinsäurebindenden Material vorbei bewegt. Die Basis, dass sich mit dem erfindungsgemäßen Mittel Nukleinsäuren aus flüssigen Proben isolieren lassen basiert dabei auf einem völlig neuartigen Prinzip. Dieses unterscheidet sich grundlegend von den bekannten Prinzipien der Isolierung von Nukleinsäuren an chromatographische Trägermaterialien. Es zeigt sich, dass es wesentlich ist, dass das für die Anbindung der Nukleinsäuren eingesetzte Material in Form von Metall-Schrauben oder Metallschwämmen oder einer Scheibe aus angerautem Polymer oder angerauter Plastgranulate eine raue Oberfläche aufweist oder dass es sich um ein oberflächenstrukturiertes Material handelt, was die Glattheit durch die Struktur an der Oberfläche (diese kann geordnet oder auch ungeordnet sein) aufhebt, wobei die Rauheit der Scheibe oder Plastgranulate durch Berühren oder durch Ansicht erkennbar ist. In Summe ist es notwendig, dass innerhalb der Pipettenspitze durch das verbrachte Material eine zwei/dreidimensionale Struktur entsteht, an welche Nukleinsäuren adsorbieren können. Die Anbindung der Nukleinsäure scheint darauf zu basieren, dass die in der Probe enthaltenen Nukleinsäuren nach Inkontaktbringen der Probe mit einer rauen Oberfläche an der rauen Oberfläche, einer strukturierten Oberfläche oder einem zwei/dreidimensionalen Netzwerk präzipitiert. Dies erfolgt dabei dadurch, dass durch die Zugabe z.B. eines Alkohols die Polarität der Umgebung geringer wird und sich dadurch die Löslichkeit der Nukleinsäure reduziert. Überraschenderweise funktioniert die "Präzipitation" der Nukleinsäure an diesen beschriebenen Oberflächen extrem effizient bei hoher Ausbeute und Reinheit.

Der Kern der Erfindung besteht somit darin, dass sich freie oder durch Lyse freigesetzte Nukleinsäuren in einer wässrigen Umgebung befinden, deren Polarität mittels organischer Substanzen so eingestellt ist, dass sich die Löslichkeit der Nukleinsäure reduziert und nachfolgend diese wässrige Umgebung in die erfindungsgemäße Pipettenspitze eingezogen wird, so dass dann die Nukleinsäure am in der Pipettenspitze verbrachten Material/Netzwerk vorbei bewegt wird (die kann durch mehrmaliges pipettieren erfolgen) und an der Oberfläche des Materials/Netzwerks präzipitiert und nachfolgend die präzipitierte DNA von der Oberfläche wieder abgelöst wird und zur Verfügung steht. Optional kann die an der Oberfläche präzipitierte Nukleinsäure auch gewaschen und nach Waschschritten abgelöst werden.

Der praktische Extraktionsablauf mittels des erfindungsgemäßen Verfahrens basiert damit auf folgenden Schritte. Nach Bereitstellung einer Nukleinsäure enthaltenen Probe in einer wässrigen Form werden die für die Präzipitation der Nukleinsäuren notwendigen Bedingungen eingestellt, dass die Nukleinsäure an das in der Pipettenspitzen verbrachte Material präzipitieren kann. Der Ansatz wird mittels Pipettiervorgänge am vertikal in der Pipettenspitze verbrachten nukleinsäurebindenden Material "vorbei pipettiert". Die Nukleinsäuren präzipitieren an das Material. Nachfolgend können optional Waschpuffer ebenfalls am nukleinsäurebindenden Material "vorbei pipettiert" werden. Danach erfolgt ein Trocknungsschritt (z.B. häufiges Auf-und Abpipettieren). Final wird das Elutionsmittel wiederum mehrmals am vertikal angeordneten nukleinsäurebindenden Material "vorbei pipettiert" und dabei die gebundene Nukleinsäure abgelöst. Die Nukleinsäure liegt nun für notwendige downstream-Applikation vor. Das Verfahren ist extrem schnell und einfach in der Durchführung und erlaubt die Isolierung von Nukleinsäuren in einer extrem hohen Ausbeute und Reinheit. Es gibt keine Probleme mit viskösen Lösungen und keine Probleme mit der Entfernung von alkoholischen Komponenten bzw. mit einer extremen Schaumbildung, wie dies alles bei der Verwendung horizontal angeordneter poröser Trägermaterialien oder bei Pipettenspitzen, die mit einem porösen chromatographischen Material befüllt sind, der Fall ist. Das Verfahren ist universell einsetzbar und kann sowohl automatisiert als auch manuell durchgeführt werden. Es ist in idealster Weise für den Einsatz einer automatisierten Nukleinsäurextraktion geeignet, da die notwendigen Schritte zur Anbindung von Nukleinsäuren, zum Waschen der gebundenen Nukleinsäuren sowie zum Ablösen der Nukleinsäuren nur noch multiple Pipettierschritte sind. Dabei umgeht die erfindungsgemäße Pipettenspitze die bekannten Nachteile aus dem Stand der Technik, welche durch die bisherige konstruktive Anordnung bzw. Befüllung von Pipettenspitzen mit porösen chromatographischen Materialien bedingt sind.

Die einzusetzenden vertikal in die Pipettenspitze verbrachten Materialien zur Anbindung von Nukleinsäuren können extrem unterschiedlich sein. Neben mineralischen Materialien können auch modifizierte Plastikmaterialien eingesetzt werden, deren Oberfläche nicht glatt, sondern rau oder strukturiert sind. Dazu zählen auch sogenannte Kompositmaterialien, die Mischungen aus Polymeren und z.B. organischen Komponenten als auch anorganische Komponenten sowie Kompositmaterialien. Wesentlich ist nur die Bereitstellung bzw. die Verbringung von Material in die Pipettenspitze, das zur Ausbildung eines zwei/dreidimensionalen Netzwerkes führt, wobei die Nukleinsäuren dann an diese Struktur präzipitieren. Die Architektur des Materials ist ebenfalls nicht limitierend (rund, rechteckig, etc.). Dabei kann es sich auch um mehrere Materialien handeln (z.B. mehrere Granulate). In einfachen Ausführungsformen kann allein schon eine in die Pipettenspitze verbrachte Schraube für die Isolierung von Nukleinsäuren eingesetzt werden.

Wichtig ist nur, dass das Material in eine Pipettenspitze verbracht wird und jederzeit von einer Flüssigkeit umspült werden kann, ohne dass die Flüssigkeit durch das eingebrachte Material hindurch muss. Auch kann eine Pipettenspitze eingesetzt werden, in welcher sich (aus einem Spritzgussteil gefertigt) dass Bindungsmaterial schon befindet und dieses nicht mehr in die Spitze verbracht werden muss. Vorteilhaft ist auch die Verwendung von rauem, magnetischen Material. Solch ein Material ist als Granulat unter dem Markennamen TECACOPM^{®} bekannt.

Der Begriff "raue Oberfläche" ist so zu verstehen, dass durch Berühren oder durch Ansicht der Oberfläche erkennbar ist, dass diese nicht glatt ist. Dabei kann es sich aber auch um eine Oberfläche handeln, die eine Struktur aufweist (z.B. Rillen). Durch diese Struktur ist die Glattheit der Oberfläche aufgehoben, auch wenn die Struktur, also die Rillen, selbst glatt sein kann. Solche Oberflächen werden als "strukturierte Oberflächen" bezeichnet. Falls durch Ansicht oder Berühren der Oberfläche nicht erkennbar ist, ob eine Oberfläche glatt oder rau ist, kann ein Test durchgeführt werden, bei dem ein Laserstrahl auf diese Oberfläche gerichtet wird. Bei einer glatten Oberfläche wird der Laser nur in Hauptrichtung an der Oberfläche reflektiert. Bei rauen Oberflächen erfolgt eine Streuung in alle Raumrichtungen. Ein solcher Test ist auf der Web-Seite der Universität Kiel beschrieben worden (http://www.tf.unikiel.de/matwis/amat/semitech_en/kap_3/illustr/oberflaechenstrukture.pdf

Die Erfindung soll nachfolgend anhand von Ausführungsbeispielen näher erläutert werden. Die Ausführungsbeispiele stellen dabei keine Limitierung der Erfindung dar.

### Ausführungsbeispiele

### Beispiel 1: Manuelle Extraktion von Nukleinsäure aus NIH 3T3 Zellen mittels des erfindungsgemäßen Verfahrens unter Verwendung einer modifizierten Pipettenspitze

In eine 1 ml Pipettenspitze (Fa. Sarstedt) wurde eine Scheibe aus Polyethylen senkrecht im letzten Drittel fixiert.

Eingesetzt wurden 5 × 10⁵ NIH 3T3 Zellen Die für die Isolierung der Nukleinsäuren eingesetzte Extraktionschemie wurde dabei teilweise aus dem kommerziellen Extraktionskit innuPREP Blood DNA Kit/IPC16X (Analytik Jena AG) genommen. Mittels eines Lysepuffers (Lysis Solution CBV) sowie von Proteinase K wurden die Zellen bei 60°C für 15 min lysiert. Die Lyse erfolgte in einem 2.0 ml Reaktionsgefäß. Nach Lyse wurde der Ansatz mit 400 µl Isopropanol versetzt. Nachfolgend wurde die modifizierte Pipettenspitze eingesetzt und mittels einer Pipette wurde der Ansatz 20 x auf- und abpipettiert. Danach wurden 3 weitere 2 ml- Reaktionsgefäße mit den alkoholischen Waschpuffern (Washing Solution LS, 80%iger Ethanol, 80%iger Ethanol) befüllt. Die Pipettenspitze wurde dann sukzessive in die jeweiligen Waschpuffer getaucht und es wurde jeweils 5 x auf- und abpipettiert. Nach dem letzten Waschschritt wurde die Spitze getrocknet und damit der restliche Ethanol entfernt. Die Elution der gebundenen Nukleinsäure erfolgte mit 100 µl Elution Buffer. Dieser wurde wiederum in ein 2 ml- Reaktionsgefäß gegeben. Es wurde 30 x auf- und abpipettiert. Nach Entfernung der Pipettenspitze liegt die isolierte Nukleinsäure im Reaktionsgefäß vor. Das Verfahren ist extrem einfach und schnell.

Der Nachweis der isolierten Nukleinsäure erfolgte mittels spektrophotometrischer Messung.

### Ergebnisse spektrophotometrische Vermessung

| | **Probe** | **Konzentration (ng/µl)** | **Ausbeute (µg)** | **Ratio A₂₆₀:A₂₈₀** | **Ratio A₂₆₀:A₂₃₀** |
|---|---|---|---|---|---|
| 1 | ca. 1 × 10⁵ NIH 3T3 Zellen | 264 | 26,4 | 1.95 | 2,10 |
| 2 | ca. 1 × 10⁵ NIH 3T3 Zellen | 228 | 22,8 | 1.94 | 2,09 |
| 3 | ca. 1 × 10⁵ NIH 3T3 Zellen | 222 | 22,2 | 1.97 | 2.11 |

Wie die Ergebnisse zeigen, ist es mit dem erfindungsgemäßen Mittel möglich, allein unter Verwendung einer Standard Extraktionschemie und mittels weniger Pipettierschritte mit einer Standardpipette Nukleinsäure zu binden und zu isolieren. Es zeigt sich, dass die Ausbeuten extrem hoch sind.

### Beispiel 2: Automatisierte Extraktion von Nukleinsäure aus NIH 3T3 Zellen mittels des erfindungsgemäßen Verfahrens und unter Verwendung einer modifizierten Pipettenspitze sowie unter Verwendung eines kommerziell verfügbaren Extraktionsautomaten

Die automatisierte Extraktion wurde mit dem Extraktionsautomaten InnuPure C16 (Analytik Jena AG) durchgeführt. Dieser Extraktionsautomat basiert auf einer Magnetpartikel-Nukleinsäureextraktion.

Für die Durchführung einer Nukleinsäureextraktion nach dem erfindungsgemäßen Verfahren wurden die Pipettenspitzen, die für den Extraktionsautomaten genutzt werden, so geändert, dass sie dem erfindungsgemäßen Mittel entsprechen. In die Pipettenspitzen wurde in das untere Drittel vertikal, eine aus einem angerauten Polymer hergestellte Scheibe eingebracht, die das Lumen nicht verschließt und somit die Pipettierfunktion der Pipettenspitzen erhalten bleibt. Es wurden dabei angeraute Scheiben aus verschiedenen Materialien eingesetzt.

Für die Nukleinsäureextraktion wurden jeweils 5×10⁵ NIH 3T3 Zellen eingesetzt. Die für die Isolierung der Nukleinsäuren eingesetzte Extraktionschemie wurde dabei teilweise aus dem kommerziellen Extraktionskit innuPREP Blood DNA Kit/IPC16X (Analytik Jena AG) genommen. Mittels eines Lysepuffers (Lysis Solution CBV) sowie von Proteinase K wurden die Zellen bei 60°C für 15 min in einem 2.0 ml Reaktionsgefäß lysiert.

Nachfolgend wurde das automatisierte Verfahren des Innupure C16 für die Aufreinigung der Nukleinsäuren verwendet. Die für die Extraktion benötigten Lösungen lagen in einer vorbefüllten Deep Well-Platte vor. Die oben beschriebenen Lysate wurden in Kavitäten gegeben, die mit 400 µl Isopropanol befüllt waren. Diese Lösung wurde daraufhin mittels der Pipettenspitze derart durchmischt, dass die Lösung seitlich an der in der Spitze eingebrachten Scheibe vorbeiströmt. Es wurden 100 Wiederholungen durchgeführt.

Danach wurde sukzessive in drei weiteren Kavitäten, die alkoholische Waschpuffer (Washing Solution LS, 80%iger Ethanol, 80%iger Ethanol) enthielten je 5x durchmischt.

Im Anschluss an den letzten Waschschritt wurde die erfindungsgemäße Spitze und die in ihr enthaltene Scheibe durch 200x Pipettieren von Luft getrocknet und damit der restliche Ethanol entfernt. Die Elution der Nukleinsäuren erfolgte durch 120x durchmischen mit 100 µl Elution Buffer, der durch das Gerät zuvor auf 50° C temperiert worden war. Das Gesamtvolumen an Elution Buffer betrug 200 µl.

Das Verfahren ist extrem einfach und schnell und zeigt, dass kommerziell verfügbare Extraktionsautomaten für die Durchführung des erfindungsgemäßen Verfahrens mit dem dazu korrespondierenden erfindungsgemäßen Mittel eingesetzt werden können. Der zeitliche Aufwand im Vergleich zu einer Magnetpartikel-basierten Extraktion ist viel geringer. Der Nachweis der isolierten Nukleinsäure erfolgte mittels spektrophotometrischer Messung.

### Ergebnisse der spektrophotometrischen Vermessung:

| **Material** | **Konzentration (ng/ µl)** | **Ausbeute (µg)** | **Ratio A₂₆₀:A₂₈₀** | **Ratio A₂₆₀:A₂₃₀** |
|---|---|---|---|---|
| Polymilchsäure | | | | |
| 1 | 31,67 | 6,34 | 1,95 | 2,19 |
| 2 | 25,18 | 5,04 | 1,8 | 2,11 |

| BioFila linen | | | | |
|---|---|---|---|---|
| 3 | 56,23 | 11,25 | 1,87 | 2,17 |
| 4 | 67,63 | 13,52 | 1,85 | 2,18 |

| Polycarbonat | | | | |
|---|---|---|---|---|
| 5 | 38,12 | 7,62 | 1,79 | 2,13 |
| 6 | 21,9 | 4,38 | 2,14 | 2,36 |

| Polyhydroxyalkanoat | | | | |
|---|---|---|---|---|
| 7 | 30,33 | 6,06 | 1,89 | 2,02 |
| 8 | 42,49 | 8,5 | 1,85 | 2,13 |

| Acryl-Nitril-Styrol | | | | |
|---|---|---|---|---|
| 9 | 27,26 | 5,45 | 1,9 | 1,87 |
| 10 | 32,03 | 6,4 | 1,76 | 2,13 |

| Polystyrol | | | | |
|---|---|---|---|---|
| 11 | 28,85 | 5,77 | 1,83 | 2,02 |
| 12 | 4,75 | 0,95 | 1,42 | 1,3 |

| Polyethylen | | | | |
|---|---|---|---|---|
| 13 | 25,43 | 5,09 | 1,76 | 1,53 |
| 14 | 48,43 | 9,96 | 1,85 | 1,82 |

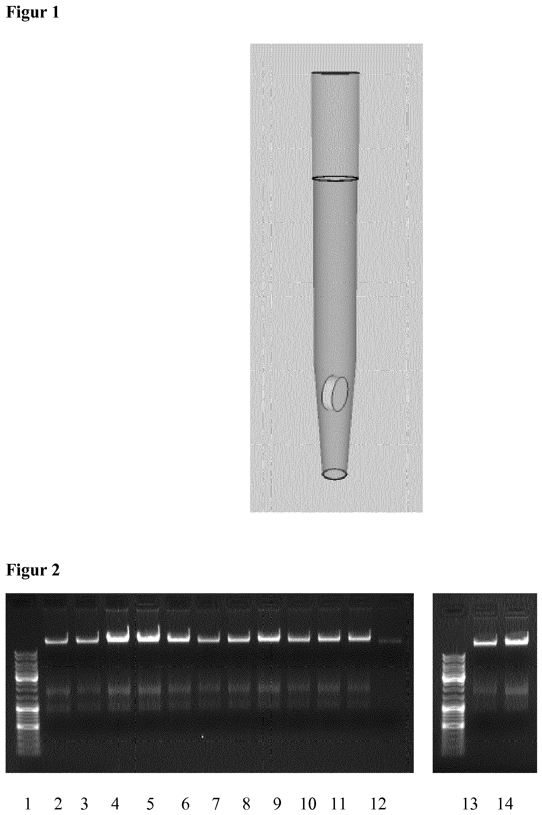

Eine gelelektrophoretische Analyse der isolierten Nukleinsäure zeigt Figur 2.

Dargestellt ist die in einem 0,8 %igen Agarosegel elektrophoretisch aufgetrennte Nukleinsäure, die mittels des erfindungsgemäßen Verfahrens isoliert wurde. Die Proben wurden von links nach rechts, beginnend mit Probe 1 aufgetragen.

Wie die Ergebnisse zeigen, ist es mit dem erfindungsgemäßen Mittel, das aus unterschiedlichen Polymeren bestehen kann, möglich, allein unter Verwendung einer Standard Extraktionschemie und mittels weniger Pipettierschritte mit einer Standard Pipettierplattform, Nukleinsäure zu Binden und zu isolieren. Es zeigt sich, dass die Ausbeuten extrem hoch sind.

### Beispiel 3: Automatisierte Extraktion von Nukleinsäure aus Blutzellen mittels des erfindungsgemäßen Verfahrens und unter Verwendung von Pipettenspitzen, welche unterschiedliche Materialien zur Isolierung von Nukleinsäuren enthalten sowie unter Verwendung eines kommerziell verfügbaren Extraktionsautomaten

Die automatisierte Extraktion wurde mit dem Extraktionsautomaten InnuPure C16 (Analytik Jena AG) durchgeführt. Dieser Extraktionsautomat basiert auf einer Magnetpartikel-Nukleinsäureextraktion.

Für die Durchführung einer Nukleinsäureextraktion nach dem erfindungsgemäßen Verfahren wurden die Pipettenspitzen, die für den Extraktionsautomaten genutzt werden, so geändert, dass sie dem erfindungsgemäßen Mittel entsprechen. Es wurden drei unterschiedliche Spitzen eingesetzt:
Typ 1: Pipettenspitze enthaltend ein dreidimensionales Netzwerk aus Metall (dazu wurde ein kommerziell verfügbarer sog. Metall Putzschwamm (Edelstahlspirale) genutzt, von welchem etwas Material abgeschnitten wurde. Dieses Material wurde in die Pipettenspitze gestopft. Es entsteht ein dreidimensionales Netzwerk, an welchem die Lösung vorbei pipettiert wird).
Typ 2: Pipettenspitze mit zwei gegeneinander gesteckten verzinkten Holzschrauben (diese stellen gemäß der Beschreibung eine strukturierte Oberfläche dar)
Typ 3: Pipettenspitze mit 4 Plastgranulaten, deren Oberfläche zuvor angeraut wurde. Diese Plastgranulate aus Polyethylen stellen gemäß der Beschreibung ein Material mit einer rauen Oberfläche dar. Ferromagnetisches Material mit Polypropylen.

Für die Nukleinsäureextraktion wurden jeweils Blutzellen aus 2 ml Vollblut, welche zuvor isoliert worden waren eingesetzt. Die Blutzellen wurden in 200 µl 1 PBS resuspensiert. Die für die Isolierung der Nukleinsäuren eingesetzte Extraktionschemie wurde dabei teilweise aus dem kommerziellen Extraktionskit innuPREP Blood DNA Kit/IPC16 (Analytik Jena AG) genommen. Der gesamte Extraktionsablauf wurde automatisiert mittels des Gerätes Innupure C16 (Analytik Jena AG) durchgeführt. Das Gerät basiert auf einem walk-away Prinzip. Dazu wird eine Deep Well Platte mit den benötigten Reagenzien vorbefüllt. Sukzessive wird dann die Pipettenspitze (mit den erfindungsgemäßen Modifikationen) in die einzelnen Kavitäten geführt und die jeweiligen Lösungen werden mittels auf- und abpipettieren in die Pipettenspitze aufgezogen und an dem in der Pipettenspitze enthaltenem Material vorbei pipettiert.

Als erstes wurde die Zellsuspension in die erste Kavität der vorbefüllten Deep Well Platte überführt. In dieser Kavität befindet sich der Lysepuffer sowie Proteinase K. Die Lyse erfolgte dabei der Art, dass das Lysat mittels der erfindungsgemäßen Pipettenspitze multiple Male auf - und abpipettiert wurde. Nach Lyse wurde das Lysat in die nächste Kavität überführt. In dieser Kavität befindet sich Isopropanol. Wiederum wurde multiple Male auf und abpipettiert und somit die Flüssigkeit permanent in das Innere der Pipettenspitze gezogen und dabei an dem in der Pipettenspitze befindlichem Material vorbei. Bei diesem Schritt bindet die Nukleinsäure an das Material. Nach diesem Schritt wurde die Pipettenspitze in die nächsten Kavitäten bewegt. In diesen befinden sich alkoholische Waschpuffer. Die gebundene Nukleinsäure wird somit wiederum durch multiple Pipettiervorgänge gewaschen. Nach Trocknung der Pipettenspitze wurde diese in eine weitere Kavität bewegt, in welcher sich Wasser befindet. Mittels auf-und abpipettieren wird die Nukleinsäure vom Material abgelöst und liegt final in gelöster Form vor. Damit ist der gesamte Extraktionsprozess vollständig automatisiert abgearbeitet.

Das Verfahren ist extrem einfach und schnell und zeigt, dass kommerziell verfügbare Extraktionsautomaten für die Durchführung des erfindungsgemäßen Verfahrens mit dem dazu korrespondierenden erfindungsgemäßen Mittel eingesetzt werden können. Der zeitliche Aufwand im Vergleich zu einer Magnetpartikel-basierten Extraktion ist viel geringer.

Der Nachweis der isolierten Nukleinsäure erfolgte mittels spektrophotometrischer Messung.

### Ergebnisse der spektrophotometrischen Vermessung:

| **Material** | **Konzentration (ng/ µl)** | **Ausbeute (µg)** | **Ratio A₂₆₀:A₂₈₀** | **Ratio A₂₆₀:A₂₃₀** |
|---|---|---|---|---|
| Spitze mit drei dimensionaler Struktur (Edelstahlwolle) | | | | |
| | 62 | 24,8 | 1,8 | 1,9 |
| 1 | 70 | 28,0 | 1,8 | 1,9 |
| 2 | 75 | 30,0 | 1,8 | 2,0 |
| 3 | | | | |

| Spitze mit verzinkten Schrauben | | | | |
|---|---|---|---|---|
| 1 | 38 | 15,2 | 1,7 | 1,6 |
| 2 | 42 | 18,0 | 1,8 | 1,8 |
| 3 | 45 | | 1,7 | 1,7 |

| Spitze mit rauem Plastgranulat aus PE | | | | |
|---|---|---|---|---|
| 1 | 80 | 32,0 | 1,8 | 2,2 |
| 2 | 94 | 37,6 | 1,8 | 2,2 |
| 3 | 102 | 40,8 | 1,8 | 2,2 |

Figur 1: zeigt eine exemplarische Darstellung der vertikal in den Hohlkörper eingebrachten Scheibe aus einem nukleinsäurebindenden Polymermaterial; dargestellt ist eine exemplarische Ausführungsform des erfindungsgemäßen Mittels, welches für die Nukleinsäureextraktion nach dem erfindungsgemäßen Verfahren eingesetzt werden kann.

## Patentansprüche

1. Vorrichtung zur Extraktion von Nukleinsäuren, umfassend einen Hohlkörper, durch den eine Flüssigkeit geleitet wird, wobei in diesem Hohlkörper ein Material so angeordnet ist, dass es von einer Flüssigkeit umspült werden kann, **dadurch gekennzeichnet, dass** es sich bei dem Material um Metall-Schrauben oder Metallschwämme oder eine Scheibe aus angerautem Polymer oder angeraute Plastgranulate handelt, wobei die Rauheit der Scheibe oder Plastgranulate durch Berühren oder durch Ansicht erkennbar ist.

2. Vorrichtung nach Anspruch 1, **dadurch gekennzeichnet, dass** es sich bei dem Hohlkörper um eine Pipettenspitze handelt und das Material eine Größe hat, die verhindert, dass es aus der Pipettenspitze austritt.

3. Vorrichtung Anspruch 1 oder 2, **dadurch gekennzeichnet, dass** es sich bei dem Hohlkörper um eine Pipettenspitze handelt, die an der Innenwand aufgeraut ist oder an deren Innenwand das eingebrachte Material immobilisiert ist.

4. Gerät nach dem walk-away-Prinzip zur automatisierten Extraktion von Nukleinsäuren, umfassend mindestens eine Vorrichtung gemäß einem der Ansprüche 1 bis 3.

5. Gerät nach Anspruch 4, **dadurch gekennzeichnet, dass** es einen Pipettierautomaten oder einen Extraktionsautomaten darstellt.

6. Verfahren zur automatisierten Extraktion von Nukleinsäuren, **gekennzeichnet durch** folgende Schritte:
a) Eine lysierte biologische Probe wird mit mindestens einer Substanz, die die Polarität der wässrigen Lösung senkt, versetzt,
b) Aufziehen dieser Mischung unter a) mit einer Pipettenspitze, in der sich ein Material gemäß einem der Ansprüche 1 bis 5 befindet und mehrmaliges Auf- und Abpipettieren, wobei sich die Flüssigkeit dabei am Material vorbei bewegt und die Nukleinsäuren an dem Material präzipitieren,
c) Entfernen der Pipettenspitze aus der Probe,
d) Eintauchen der Pipettenspitze in eine Waschpufferlösung und mehrmaliges Auf- und Abpipettieren, wobei sich die Flüssigkeit dabei am Material vorbei bewegt.
e) Trocknung der Pipettenspitze,
f) Ablösen der Nukleinsäure mit einem Elutionspuffer durch mehrmaliges Auf- und Abpipettieren des Elutionspuffers, wobei sich der Elutionspuffer dabei am Material vorbei bewegt.

7. Verfahren nach Anspruch 6, **dadurch gekennzeichnet, dass** als Substanz zur Senkung der Polarität der wässrigen Lösung organische Lösungsmittel, vorzugsweise Alkohole, eingesetzt werden.

8. Verfahren gemäß Anspruch 6 mittels eines Pipettierautomaten oder einen Extraktionsautomaten.

## Claims

1. A device for extracting nucleic acids, comprising a hollow body, through which a fluid is guided, wherein a material is arranged in said hollow body such that the material can be washed around with a fluid, **characterized in that** the material are metal screws or metal sponges or a disk made out of roughened polymer or roughened plastic granules, wherein the roughness of the disk or of the plastic granules is recognizable by touch or view.

2. The device according to patent claim 1, **characterized in that** the hollow body is a pipette tip, and the material is of a size that prevents it from leaking from the pipette tip.

3. The device according to patent claim 1 or 2, **characterized in that** the hollow body is a pipette tip, which is roughened on the inner wall, or on the inner wall of which the material introduced is immobilized.

4. An apparatus according to the walk-away principle for automated extraction of nucleic acids, comprising at least a device according to any one of patent claims 1 to 3.

5. The apparatus according to patent claim 4, **characterized in that** it constitutes an automatic pipetting system or an automatic extraction system.

6. A method for automated extraction of nucleic acids, **characterized by** the following steps :
a) At least one substance, which lowers the polarity of the aqueous solution, is added to a lysed biological sample
b) Drawing said mixture in a) into a pipette tip containing a material according to any of patent claims 1 to 5, and pipetting and pipetting off several times, wherein the fluid is moving past the material in the process, and the nucleic acids precipitate on the material
c) Removing the pipette tip from the sample
d) Immersing the pipette tip into a wash buffer solution and pipetting and pipetting off several times, wherein the fluid is moving past the material in the process
e) Drying the pipette tip
f) Detaching the nucleic acid by means of an elution buffer by pipetting and pipetting off the elution buffer several times, wherein the elution buffer is moving past the material in the process.

7. The method according to patent claim 6, **characterized in that** organic solvents, preferably alcohols, are used as a substance for lowering the polarity of the aqueous solution.

8. The method according to patent claim 6 by means of an automatic pipetting system or an automatic extraction system.

## Revendications

1. Dispositif d'extraction d'acides nucléiques, comportant un corps creux par lequel un liquide est conduit, dans lequel une matière est disposée dans ce corps creux de manière à pouvoir être rincée par un liquide, **caractérisé en ce que** la matière sont des vis métalliques ou des éponges métalliques ou un disque de polymère rugueux ou des granulés de plastique rugueux, dans lequel la rugosité du disque ou des granulés de plastique est reconnaissable au toucher ou à la vue.

2. Dispositif selon la revendication 1, **caractérisé en ce que** le corps creux est une pointe de pipette, et la matière a une taille, qui l'empêche de sortir de la pointe de pipette.

3. Dispositif selon la revendication 1 ou 2, **caractérisé en ce que** le corps creux est une pointe de pipette , qui est rugueuse sur sa paroi interne ou sur la paroi interne de laquelle la matière introduite est immobilisée.

4. Appareil selon le principe *walk-away* pour l'extraction automatisée des acides nucléiques, comportant au moins un dispositif selon l'une quelconque des revendications 1 à 3.

5. Appareil selon la revendication 4, **caractérisé en ce qu'**il représente un appareil automatique de pipetage ou un appareil automatique d'extraction.

6. Procédé d'extraction automatique des acides nucléiques, **caractérisé par** les étapes suivantes :
a) au moins une substance, qui abaisse la polarité de la solution aqueuse, est ajoutée à un échantillon biologique lysé
b) aspirer ce mélange sous a) avec une pointe de pipette, dans laquelle se trouve une matière selon l'une quelconque des revendications 1 à 5, et l'aspirer et la pipeter plusieurs fois, dans lequel le liquide passe alors devant la matière, et les acides nucléiques précipitent dans la matière
c) retirer la pointe de pipette de l'échantillon
d) plonger la pointe de pipette dans une solution tampon de lavage, et l'aspirer et la pipeter plusieurs fois, dans lequel le liquide passe alors devant la matière
e) séchage de la pointe de pipette
f) détachement de l'acide nucléique à l'aide d'un tampon d'élution en aspirant et pipettant plusieurs fois le tampon d'élution, dans lequel le tampon d'élution passe alors devant la matière.

7. Procédé selon la revendication 6, **caractérisé en ce que** des solvants organiques, de préférence des alcools, sont utilisés comme substance pour abaisser la polarité de la solution aqueuse.

8. Procédé selon la revendication 6 à l'aide d'un appareil automatique de pipetage ou d'un appareil automatique d'extraction.
